(19)

**Europäisches Patentamt**
**European Patent Office**
**Office européen des brevets**

(11) **EP 2 982 364 B1**

(12) **EUROPEAN PATENT SPECIFICATION**

(45) Date of publication and mention
of the grant of the patent:
**12.02.2020 Bulletin 2020/07**

(21) Application number: **14779760.9**

(22) Date of filing: **25.03.2014**

(51) Int Cl.:
*A61K 8/31* (2006.01)    *A61K 8/06* (2006.01)
*A61K 8/37* (2006.01)    *A61K 8/67* (2006.01)
*A61Q 19/00* (2006.01)   *A61K 9/00* (2006.01)
*A61K 47/14* (2017.01)   *A61K 8/35* (2006.01)
*A61K 9/107* (2006.01)

(86) International application number:
**PCT/JP2014/058358**

(87) International publication number:
**WO 2014/162930 (09.10.2014 Gazette 2014/41)**

(54) **OIL-IN-WATER EMULSION COMPOSITION**

ÖL-IN-WASSER-EMULSIONSZUBEREITUNG

COMPOSITION DE TYPE ÉMULSION HUILE-DANS-EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.04.2013 JP 2013079824**

(43) Date of publication of application:
**10.02.2016 Bulletin 2016/06**

(73) Proprietor: **FUJIFILM Corporation
Tokyo 106-8620 (JP)**

(72) Inventors:
• **KITAOKA, Hiroyuki
Ashigarakami-gun
Kanagawa 258-8577 (JP)**
• **MORI, Mikinaga
Ashigarakami-gun
Kanagawa 258-8577 (JP)**

(74) Representative: **Klunker IP
Patentanwälte PartG mbB
Destouchesstraße 68
80796 München (DE)**

(56) References cited:
EP-A1- 1 864 578         EP-A1- 2 570 112
WO-A1-2012/133246        DE-A1-102004 003 478
JP-A- H0 344 322         JP-A- H0 344 322
JP-A- H06 271 421        JP-A- H09 301 847
JP-A- H10 101 524        JP-A- 2002 193 790
JP-A- 2004 524 395       JP-A- 2007 518 757
JP-A- 2008 169 208       JP-A- 2012 505 155
US-A1- 2005 037 115      US-A1- 2010 247 504

• **MANABU OGAWA ET AL:** "Development of
Astaxanthin Nano Emulsion with Improved Shelf
Life and Enhanced Absorbability", FUJIFILM
RESEARCH & DEVELOPMENT, vol. 52, 1 January
2007 (2007-01-01), pages 26-29, XP055227758,
• **TOMOKO TASHIRO:** 'Tokushu Hada to Kobunshi
Development of Astaxanthin Nano Emulsion'
KOBUNSHI vol. 58, 2009, XP008178093
• **MANABU OGAWA ET AL.:** 'Development of
astaxanthin nano emulsion with improved shelf
life and enhanced absorbability' FUJIFILM
RESEARCH & DEVELOPMENT vol. 52, 2007,
pages 26 - 29, XP055227758
• **YOSHISADA NAKAMURA:** 'ASTALIFT'
Functional Cosmetics Containing Astaxanthin
Nano Emulsion' MEMBRANE vol. 34, no. 2, 2009,
pages 104 - 106, XP008178097

**Description**

**BACKGROUND OF THE INVENTION**

1. Field of the Invention

The present invention relates to an oil-in-water emulsion composition.

2. Description of the Related Art

In recent years, the high functionality of an oil-soluble antioxidant such as a carotenoid or a tocotrienol has drawn attention, and various compositions containing the oil-soluble antioxidant have been suggested.

JP4717790B describes a technique for improving heat resistance of a carotenoid in a preparation containing hydrogenated oil, an emulsifier, a polyol, and water.

JP2011-241177A describes a carotenoid-containing composition obtained by heating a liquid of an oil phase component mixture, which contains a carotenoid component containing a crystalline carotenoid, a specific (poly) glycerin fatty acid ester, and ascorbic acid, under a specific heating condition.

JP2002-78447A describes a technique for stabilizing a milk beverage by concurrently using lycopene, ascorbic acid, and tocopherol.

**SUMMARY OF THE INVENTION**

Particularly, among carotenoids as oil-soluble antioxidants, lycopene is a compound which is easily oxidized and decomposed and poor in stability. Therefore, if preserved for a long period of time, the compound is easily oxidized and decomposed in some cases.

As one of the methods of adding an oil-soluble component such as an oil-soluble antioxidant to an aqueous composition, there is a method of adding the oil-soluble component to an oil phase (emulsion particles) of an oil-in-water emulsion composition.

However, if the oil-in-water emulsion composition, which contains the easily oxidized and decomposed oil-soluble antioxidant such as a carotenoid, is preserved for a long period of time, the oxidation and decomposition of the oil-soluble antioxidant easily occur, and thus the effect expected to be obtained by the addition of the oil-soluble antioxidant tends to be reduced. From the viewpoints of the transparency of the composition, the permeability of the composition into the skin, and the like, it is desired that the particle size of the emulsion particles is small in some cases. However, the oxidation and decomposition of the easily oxidized and decomposed component such as a carotenoid more markedly occurs when the particle size of the emulsion particles is small.

The present invention has been made in consideration of the aforementioned current circumstances, and on object thereof is to provide an oil-in-water emulsion composition in which the decomposition of an oil-soluble antioxidant is inhibited and which is excellent in preservation stability.

The present invention is defined in the appended claims and shown below.

[1] An oil-in-water emulsion composition comprising:

an oil phase containing octyldodecyl myristate as an oil having an I/O value of equal to or less than 0.15 and lycopene as an oil-soluble antioxidant; a water phase containing at least one kind of compound selected from the group consisting of ascorbic acid, a derivative thereof selected from the group consisting of sodium L-ascorbate, potassium L-ascorbate, calcium L-ascorbate, L-ascorbic acid phosphoric acid ester, a magnesium salt of L-ascorbic acid phosphoric acid ester, L-ascorbic acid sulfuric acid ester, a disodium salt of L-ascorbic acid sulfuric acid ester, L-ascorbic acid stearic acid ester, L-ascorbic acid 2-glucoside, L-ascorbic acid palmitic acid ester, L-ascorbyl tetraisopalmitate, and fatty acid esters of ascorbic acid such as L-ascorbyl stearic acid ester, L-ascorbyl tetraisopalmitic acid ester, and L-ascorbyl palmitic acid ester, and a salt of these as a water-soluble antioxidant, and a polyglycerin fatty acid ester having HLB of equal to or greater than 10 as an emulsifier, wherein the polyglycerin fatty acid ester having HLB of equal to or greater than 10 comprises at least one compound selected from the group consisting of decaglycerin monooleic acid ester, decaglycerin monostearic acid ester, decaglycerin monopalmitic acid ester, decaglycerin monomyristic acid ester and decaglycerin monolauric acid ester, wherein a content of the octyldocecyl myristate in the oil phase is in a range of 85% by mass to 99% by mass, in which the average particle size of emulsion particles is equal to or less than 120 nm.

[2] The emulsion composition described in [1], in which the lycopene is contained in an amorphous state.

According to the present invention, it is possible to provide an oil-in-water emulsion composition in which the decomposition of an oil-soluble antioxidant is inhibited and which is excellent in preservation stability.

## DESCRIPTION OF THE PREFERRED EMBODIMENTS

The oil-in-water emulsion composition of the present invention (hereinafter, referred to as an "emulsion composition of the present invention" as appropriate) is an oil-in-water emulsion composition containing an oil phase, which contains oil having an I/O value of equal to or less than 0.15 and an oil-soluble antioxidant, and a water phase which contains a water-soluble antioxidant. In the oil-in-water emulsion composition, the average particle size of the emulsion particles is equal to or less than 120 nm.

In the emulsion composition of the present invention, the decomposition of the oil-soluble antioxidant is inhibited. Accordingly, the emulsion composition can maintain high functionality over a long period of time.

In an emulsion composition which contains the oil-soluble antioxidant in the emulsion particles as the oil phase, the chance of contact between the oil-soluble antioxidant and an active oxidation species increases over time. As a result, the oil-soluble antioxidant is oxidized and decomposed, and thus the preservation stability of the emulsion composition tends to deteriorate. Such a tendency becomes stronger as the particle size of the emulsion particles is reduced.

In contrast, in the emulsion composition of the present invention, the oil having a specific I/O value and the oil-soluble antioxidant are contained in the oil-phase in combination, the water-soluble antioxidant is contained in the water phase, and the average particle size of the emulsion particles is 120 nm. Therefore, the oxidation and decomposition of the oil-soluble antioxidant is effectively inhibited, and the long-term preservation stability of the emulsion composition is greatly improved. The effect of inhibiting the decomposition of the oil-soluble antioxidant in the emulsion composition of the present invention is not sufficiently exhibited if any one of the elements, which include the oil having a specific I/O value that is contained in the oil phase, the water-soluble antioxidant contained in the water phase, and setting the average particle size of the emulsion particles to be equal to or less than 120 nm, is missed.

Presumably, in the emulsion composition of the present invention, the effect of inhibiting the decomposition of the oil-soluble antioxidant is obtained for the following reason. Even when the oil-soluble antioxidant becomes a radical by being oxidized in the oil phase (emulsion particles), due to the interaction between the oil having a specific I/O value contained in the oil phase and the water-soluble antioxidant contained in the water phase in the interface between the oil phase and the water phase, the oil-soluble antioxidant which has become a radical is efficiently reduced. However, the present invention is not limited to the presumption.

The emulsion composition of the present invention is an oil-in-water emulsion composition and is preferably obtained by emulsifying and mixing together a water phase composition constituted with water phase components and an oil phase composition constituted with oil phase components.

In the emulsion composition of the present invention, the emulsion particles are present as an oil phase (dispersed phase) in the emulsion composition of the present invention that is an oil-in-water type.

In the present invention, the "water phase" is used as a term contrast to the "oil phase" regardless of the type of a solvent.

In the present specification, the term "step" includes not only an independent step but also a step which cannot be clearly differentiated from other steps as long as the intended object of the step is achieved.

In the present specification, the range of numerical values described by using "to" represents a range which includes numerical values listed before and after "to" as a minimum value and a maximum value, respectively.

In the present specification, when there is a plurality of substances corresponding to each of the components in the composition, unless otherwise specified, the amount of each of the components in the composition means the total amount of the plurality of substances present in the composition.

In the present invention, the "preservation stability" means a property in which the decomposition of the oil-soluble antioxidant contained in the emulsion composition is inhibited, and the intended effect expected to be obtained as a result of adding the oil-soluble antioxidant to the emulsion composition can be maintained over a long period of time, even after the emulsion composition is preserved for a long period of time.

Hereinafter, each of the constituents in the present invention will be more specifically described.

(Oil having I/O value of equal to or less than 0.15)

The emulsion composition of the present invention contains oil having an I/O value of equal to or less than 0.15 (hereinafter, referred to as a "specific oil" as appropriate) in an oil phase.

As the specific oil in the present invention, octyldocecyl myristate is used.

Octyldocecyl myristate may be used singly, or in combination with one or more kinds thereof.

The I/O value is a parameter as an index showing a degree of hydrophilicity and hydrophobicity of a compound by using a ratio of an inorganic group to an organic group. "I" represents inorganicity, and "O" represents organicity. The greater the I/O value, the higher the inorganicity. The I/O value is specifically described in "Organic Conceptual Diagram (Koda Yoshio, SANKYO PUBLISHING Co., Ltd., 1984).

The concept of the I/O value is defined by classifying the properties of compounds into organic groups that form a covalent bond and inorganic groups that form an ionic bond and positioning every single organic compound in each point on rectangular coordinates having axes named an organic axis and an inorganic axis.

A value of inorganicity is a value obtained by digitizing the extent of an influence of various substituents, bonds, and the like of an organic compound on a boiling point based on a hydroxyl group. Moreover, a value of organicity is a value determined based on an influence of a carbon atom, which represents a methylene group, on a boiling point by using the methylene group in a molecule as a unit.

The closer the I/O value is to 0, the closer the organic compound is to a non-polar compound (having high hydrophobicity and organicity), and the greater the I/O value is, the closer the organic compound is to a polar compound (having high hydrophilicity and inorganicity).

The standard values of the organicity (O value) and the inorganicity (I value) used for calculating the I/O value in the present invention are shown in the following Table 1.

[Table 1]

|  | O value | I value |
|---|---|---|
| Number of carbon atoms in molecule | 20 | 0 |
| Carbon-carbon double bond | 0 | 2 |
| Carbon-carbon triple bond | 0 | 3 |
| Number of branches of carbon chain | -10 | 0 |
| COO | 0 | 60 |
| -OH | 0 | 100 |
| -O - | 0 | 20 |

The I/O value of the specific oil is equal to or greater than 0 and equal to or less than 0.15. From the viewpoint of inhibiting the decomposition of the oil-soluble antioxidant, the I/O value is preferably equal to or greater than 0.05 and equal to or less than 0.13, and more preferably equal to or greater than 0.06 and equal to or less than 0.11.

In the present invention, the "oil" is defined as a compound which dissolves in an amount of equal to or less than 0.1% by mass in water at 25°C and 1 atm, contains an alkyl group having 4 or more carbon atoms in a molecule, and does not have a hydrophilic group such as a hydroxyl group or a polyether group.

Among the compounds included in the specific oil, from the viewpoint of emulsifiability and from the viewpoint of inhibiting the decomposition of the oil-soluble antioxidant, a monoester compound is preferable in which a fatty acid and a monohydric alcohol are condensed with each other.

As the fatty acid constituting the monoester compound, a fatty acid having 4 to 30 carbon atoms (more preferably having 6 to 24 carbon atoms, and even more preferably having 8 to 22 carbon atoms) is preferable. Examples of such a fatty acid include butyric acid, valeric acid, caproic acid, caprylic acid, capric acid, lauric acid, myristic acid, pentadecylic acid, palmitic acid, margaric acid, stearic acid, oleic acid, isostearic acid, arachidic acid, ethyl and hexanoic acid.

As the monohydric alcohol condensed with the fatty acid, a monohydric alcohol having 1 to 40 carbon atoms (more preferably having 2 to 30 carbon atoms, and even more preferably having 3 to 24 carbon atoms) is preferable, and examples thereof include methanol, ethanol, propanol, isopropanol, butanol, pentanol, hexanol, capryl alcohol, lauryl alcohol, myristyl alcohol, cetyl alcohol, stearyl alcohol, isostearyl alcohol, oleyl alcohol, behenyl alcohol, and 2-octyldodecanol.

The monoester compound used as the specific oil is preferably a compound in which the fatty acid and the monohydric alcohol are condensed with each other so as to form a combination having an I/O value of equal to or less than 0.15.

The specific oil includes octyldodecyl myristate and optionally cetyl ethylhexanoate, isopropyl isostearate, isostearyl isostearate, dialkyl carbonate (C14, C15), and liquid paraffin. Furthermore, as the specific oil, commercially available products may be used.

From the viewpoint of inhibiting the oxidation and decomposition of the oil-soluble antioxidant, the content of the specific oil is 85% by mass to 99% by mass, and preferably 90% by mass to 99% by mass, with respect to the total mass of the components contained in the oil phase.

The content of the specific oil in the emulsion composition of the present invention is preferably 0.0001% by mass to 40% by mass, more preferably 0.001% by mass to 30% by mass, and even more preferably 0.01% by mass to 25% by mass, with respect to the total mass of the emulsion composition.

From the viewpoint of inhibiting the oxidation and decomposition of the oil-soluble antioxidant and from the viewpoint of emulsifiability, a ratio between the content of the specific oil and the content of the oil-soluble antioxidant (specific oil:oil-soluble antioxidant) is preferably 1:1 to 10,000:1, more preferably 1:1 to 1,000:1, and even more preferably 3:1 to 200:1, based on mass.

From the viewpoint of inhibiting the oxidation and decomposition of the oil-soluble antioxidant, a ratio between the content of the specific oil and the content of the water-soluble antioxidant (specific oil:water-soluble antioxidant) is preferably 30:1 to 1:10,000, more preferably 20:1 to 1:1,000, and even more preferably 10:1 to 1:200, based on mass.

<Oil-soluble antioxidant>

The emulsion composition of the present invention contains at least one kind of oil-soluble antioxidant.

The oil-soluble antioxidant in the present invention is contained in the emulsion composition, as one of the constituents of the oil phase (emulsion particles).

The emulsion composition of the present invention may contain one kind of the oil-soluble antioxidant singly or two or more kinds thereof concurrently.

In the present invention, the "oil-soluble antioxidant" may be a compound, which dissolves in an amount of equal to or less than 0.1% by mass in water at 25°C and 1 atm and functions as an antioxidant, among various antioxidants described in "Theory and Practice of Antioxidants" (Kajimoto, Sanshobo, 1984) and "Handbook of Antioxidants" (Saruwatari, Nishino, Tabata, TAISEISHA, LTD., 1976) and carotenoids which will be described later.

The oil phase contains lycopene as an oil-soluble antioxidant. Examples of further preferred oil-soluble antioxidants in the present invention include a phenol-based antioxidant, vitamin E and a carotenoid. Among these oil-soluble antioxidants, a carotenoid such as lycopene or astaxanthin and vitamin E such as tocotrienol are oil-soluble antioxidants that are particularly easily oxidized and decomposed as the preservation period of the emulsion composition is lengthened. However, in the emulsion composition of the present invention, even when these easily oxidized and decomposed antioxidants are used, the effect, which is expected to be exerted as a result of adding the oil-soluble antioxidant to the emulsion composition, can be maintained over a long period of time.

-Phenol-based antioxidant-

Examples of the phenol-based antioxidant include a compound selected from aromatic carboxylic acids, cinnamic acids, ellagic acids, BHT (butyl hydroxy toluene), BHA (butyl hydroxy anisole), and vitamin E.

Examples of the aromatic carboxylic acids include gallic acid (3,4,5-hydroxybenzoic acid) and derivatives thereof. Examples of the derivatives of the gallic acid (3,4,5-hydroxybenzoic acid) include gallic acid ester such as propyl gallate, epicatechin gallate, and epigallocatechin gallate and gallic acid glycoside such as gallotannin.

Examples of the cinnamic acids include ferulic acid, chlorogenic acid, and derivatives of these. Examples of the derivatives of ferulic acid and chlorogenic acid include ferulic acid ester. Specifically, examples thereof include ferulic acid, γ-oryzanol (rice bran extract), coffeic acid (caffeic acid or 3,4-dihydroxycinnamic acid), chlorogenic acid, glyceryl ferulate and dihydroferulic acid.

Examples of the ellagic acids include ellagic acid.

-Vitamin E~

The vitamin E is not particularly limited, and examples thereof include a compound selected from the group of compounds consisting of tocopherol and derivatives thereof and the group of compounds consisting of tocotrienol and derivatives thereof. One kind of the vitamin E may be used singly, or a plurality of kinds of the vitamin E may be used concurrently. Furthermore, as the vitamin E, a compound selected from the group of compounds consisting of tocopherol and derivatives thereof and a compound selected from the group of compounds consisting of tocotrienol and derivative thereof may be used in combination.

The group of compounds consisting of tocopherol and derivatives thereof includes dl-α-tocopherol, dl-β-tocopherol, dl-γ-tocopherol, dl-δ-tocopherol, dl-α-tocopherol acetate, dl-α-tocopherol nicotinate, dl-α-tocopherol linoleate, dl-α-tocopherol succinate. Among these, dl-α-tocopherol, dl-β-tocopherol, dl-γ-tocopherol, dl-δ-tocopherol, and a mixture (mixed tocopherol) of these are more preferable. Furthermore, as the tocopherol derivatives, carboxylic acid esters of these, particularly, acetic acid esters of these are preferably used.

The group of compounds consisting of tocotrienol and derivatives thereof includes α-tocotrienol, β-tocotrienol, γ-tocotrienol, and δ-tocotrienol. Furthermore, as the tocotrienol derivatives, carboxylic acid esters of these, particularly, acetic acid esters of these, are preferably used.

-Carotenoid-

The emulsion composition of the present invention contains lycopene, which is a carotenoid, as the embodiment of the oil-soluble antioxidant.

In the present invention, one kind of carotenoid may be used singly, or two or more kinds thereof may be used concurrently.

The carotenoid is a pigment of terpenoids ranging from yellow to red in color, and examples thereof include a carotenoid derived from plants, algae, and bacteria. The carotenoid is not limited to a naturally occurring carotenoid, and any type of carotenoid may be used as long as it is obtained according to common methods.

Further examples of the carotenoid include α-carotene, β-carotene, γ-carotene, δ-carotene, actinioerythrol, bixin, canthaxanthin, capsorubin, β-8'-apo-carotenal (apocarotenal), β-12'-apo-carotenal, xanthophylls (for example, astaxanthin, fucoxanthin, lutein, zeaxanthin, capsanthin, β-cryptoxanthin, and violaxanthin), and hydroxyl or carboxyl derivatives of these. Lycopene may be used singly, or in combination with two or more kinds of the carotenoids in combination.

The carotenoid in the present invention is a crystalline carotenoid. The "crystalline carotenoid" does not mean a specific carotenoid. It means carotenoid which can be present as crystals at any temperature within a temperature range of -5°C to 35°C due to various factors such as a manufacturing method thereof, treatment, and preservation when the carotenoid is in the form of oil or paste containing the carotenoid. Particularly, lycopene, which will be described later, β-carotene, δ-carotene, zeaxanthin, lutein and astaxanthin are carotenoids that are easily present as crystals.

The emulsion composition of the present invention contains lycopene among the carotenoids.

The lycopene is a carotenoid represented by a chemical formula $C_{40}H_{56}$ (molecular weight: 536.87). It is a red pigment which belongs to carotenes as a type of carotenoid and exhibits absorption maximum at 474 nm (acetone).

The lycopene is known to have an extremely strong antioxidative effect and whitening effect. Therefore, conventionally, the addition of the lycopene to raw materials of foods, cosmetics, and medicines and to processed products thereof is desired, examined, and performed.

The lycopene also includes a cis isomer and a trans isomer having conjugated double bond in the center of the molecule. Examples of the isomers include an all-trans isomer, a 9-cis isomer and a 13-cis isomer. In the present invention, any of these isomers may be used.

The lycopene may be used for preparing the emulsion composition of the present invention, in the form of lycopene-containing oil or lycopene-containing paste separated and extracted from natural substances containing lycopene.

In the nature, the lycopene is contained in tomatoes, persimmons, watermelons and pink grapefruits. The lycopene-containing oil or the lycopene-containing paste may be separated and extracted from these nature substances. As forms of products containing lycopene, 4 types such as an oil type, an emulsion type, a paste type, and a powder type are known. The lycopene used in the present invention may be an extracted from natural substances or may be a substance obtained by appropriately purifying the extract if necessary. The lycopene used in the present invention may also be a synthesized product.

As one of the preferred forms of the lycopene used in the present invention, tomato-derived lycopene is exemplified. Examples of the tomato-derived lycopene include lycopene contained in an oil-soluble extract extracted from tomato pulp. In view of stability, quality, and productivity, the lycopene contained in the oil-soluble extract extracted from tomato pulp is particularly preferable. The oil-soluble extract extracted from tomato pulp means an extract extracted from pulp-like solids that is obtained by performing centrifugation on a ground substance obtained by grinding tomatoes by using an oleaginous solvent.

As the oil-soluble extract extracted from tomato pulp, it is possible to use commercially available tomato extracts widely marketed as lycopene-containing oil or lycopene-containing paste. Examples of the commercially available products include Lyc-O-Mato 80%, Lyc-O-Mato 15%, and Lyc-O-Mato 6% distributed by Sun Bright Co., Ltd., Lycopene 18 distributed by KYOWA HAKKO BIO CO., LTD ..

In the present invention, the lycopene is preferably contained in the emulsion composition of the present invention in an amorphous state. One of the preferred embodiments of the emulsion composition of the present invention is an embodiment in which the emulsion composition contains lycopene in an amorphous state as carotenoid. In many cases, the stability of the amorphous state is poorer than that of a crystalline state. However, in the emulsion composition of the present invention, by changing the crystalline state of the carotenoid into the amorphous state, the stability is further improved. Furthermore, if the carotenoid is in the amorphous state, when the emulsion composition is used for a living body, the carotenoid can pass through the skin better or can be absorbed better into the body.

The state in which the crystalline carotenoid is in the amorphous state may be confirmed by using known means for detecting a crystal structure. Furthermore, whether the carotenoid is the crystalline carotenoid may be confirmed by common methods. For example, it is possible to use differential scanning calorimetry (DSC), polarized light microscopy, X-ray diffraction (XRD). If crystals cannot be detected by these known techniques, the carotenoid can be regarded as being amorphous. Particularly, in the present invention, it is preferable to confirm whether the carotenoid is amorphous, based on the presence of an endothermic peak in DSC. Specifically, by using DSC Q2000 (TA Instruments Japan), a sample is freeze-dried to remove moisture, and in this state, an endothermic temperature and an exothermic temperature are measured in one cycle of heating-cooling (15°C/min) within a temperature range of 30°C to 200°C. If the presence of a recognizable endothermic peak is not confirmed, the carotenoid is regarded as being in an amorphous state.

Regarding the carotenoid contained in the emulsion composition of the present invention, in view of stability and dynamic absorption properties of the carotenoid, at least 50% by mass to 100% by mass of the crystalline carotenoid is preferably amorphous, 90% by mass to 100% by mass of the crystalline carotenoid is more preferably amorphous, and 95% by mass to 100% by mass of the crystalline carotenoid is even more preferably amorphous.

The state in which the carotenoid contains at least 50% by mass of the crystalline carotenoid in an amorphous state can be confirmed by, for example, comparing the amount of absorbed heat, which is indicated by the endothermic peak resulting from the carotenoid crystal in the composition of the present invention that is measured by differential scanning calorimetry (DSC), with the amount of absorbed heat which is indicated by the endothermic peak of a carotenoid crystal sample.

In addition, by comparing a spectrum of the emulsion composition of the present invention in X-ray diffraction with a spectrum of the carotenoid crystal sample, the state in which the carotenoid contains at least 50% by mass of the crystalline carotenoid in an amorphous state can be confirmed.

Furthermore, by using a crystalline carotenoid reagent that can be purchased as a commercially available product and by regarding the content thereof as being 100%, a DSC peak area is determined or X-ray diffraction (XRD) is performed, and the obtained result can be converted into the content of the crystalline carotenoid in an amorphous state. Examples of the commercially available product of the crystalline carotenoid reagent include reagents for biochemistry that can be purchased from Wako Pure Chemical Industries, Ltd..

The crystalline carotenoid may singly constitute the carotenoid in the present invention or may constitute the carotenoid in the present invention together with oil used at the time of extracting the carotenoid from natural substances.

The carotenoid in the present invention may contain an amorphous carotenoid derived from the nature, in addition to the aforementioned crystalline carotenoid.

The content of the oil-soluble antioxidant is preferably 0.01% by mass to 40% by mass, more preferably 0.05% by mass to 35% by mass, and even more preferably 0.1% by mass to 30% by mass, with respect to the total mass of the components contained in the oil phase.

The content of the oil-soluble antioxidant in the emulsion composition of the present invention is preferably 0.00001% by mass to 10% by mass, more preferably 0.00005% by mass to 5% by mass, and even more preferably 0.0001% by mass to 3% by mass, with respect to the total mass of the emulsion composition.

When the emulsion composition of the present invention contains a carotenoid as the oil-soluble antioxidant, from the viewpoint of making the carotenoid perform the function as expected, the content of the carotenoid is preferably 0.01% by mass to 5% by mass, more preferably 0.05% by mass to 3% by mass, and even more preferably 0.1% by mass to 2% by mass, with respect to the total mass of the components contained in the oil phase.

When the emulsion composition of the present invention contains a carotenoid as the oil-soluble antioxidant, the content of the carotenoid is preferably 0.00001 % by mass to 1% by mass, more preferably 0.00005% by mass to 0.8% by mass, and even more preferably 0.0001% by mass to 0.1% by mass, with respect to the total mass of the emulsion composition. If the content of the carotenoid in the emulsion composition is equal to or greater than 0.00001% by mass, the carotenoid is expected to be able to perform the intended function, and if the content of the carotenoid is 1% by mass, better preservation stability can be exhibited.

(Water-soluble antioxidant)

The emulsion composition of the present invention contains at least one kind of water-soluble antioxidant in the water phase.

In the present invention, one kind of the water-soluble antioxidant may be used singly, or two or more kinds thereof may be used concurrently.

In the present invention, the water-soluble antioxidant is defined as a compound which has an ability to prevent oxidation and dissolves in an amount of equal to or greater than 0.3% by mass in water at 25°C.

As the water-soluble antioxidant, for example, known water-soluble antioxidants can be used. The water-soluble antioxidants include the group of compounds consisting of ascorbic acid, a derivative thereof, a salt of these (hereinafter, referred to as an "ascorbic acid compound" as appropriate). As a further water-soluble antioxidant erythorbic acid, a derivative thereof, a salt of these, polyphenols, may be included.

The ascorbic acid may be any of an L-isomer, a D-isomer, and a DL-isomer. However, from the view point of availability, an L-isomer is preferable. Furthermore, a water-soluble ascorbic acid compound is preferable.

Examples of the ascorbic acid compound include sodium L-ascorbate, potassium L-ascorbate, calcium L-ascorbate, L-ascorbic acid phosphoric acid ester, a magnesium salt of L-ascorbic acid phosphoric acid ester, L-ascorbic acid sulfuric acid ester, a disodium salt of L-ascorbic acid sulfuric acid ester, L-ascorbic acid stearic acid ester, L-ascorbic acid 2-glucoside, L-ascorbic acid palmitic acid ester, L-ascorbyl tetraisopalmitate, and fatty acid esters of ascorbic acid such as L-ascorbyl stearic acid ester, L-ascorbyl tetraisopalmitic acid ester, and L-ascorbyl palmitic acid ester, and the like.

Among the aforementioned group of ascorbic acid compounds, from the viewpoint of inhibiting the decomposition of the carotenoid and from the viewpoint of preservation stability, L-ascorbic acid, sodium L-ascorbate, potassium L-ascorbate, a magnesium salt of L-ascorbic acid phosphoric acid ester, or a disodium salt of L-ascorbic acid sulfuric acid ester is preferable.

From the viewpoint of obtaining a sufficient effect with a small amount of the ascorbic acid compound, in one of the particularly preferred embodiments of the ascorbic acid compound, ascorbic acid, sodium ascorbate, or a combination of these is used.

Examples of the erythorbic acid, a derivative thereof, and a salt of these include erythorbic acid, sodium erythorbate, potassium erythorbate, calcium erythorbate, erythorbic acid phosphoric acid ester, and erythorbic acid sulfuric acid ester.

Examples of the group of compounds consisting of polyphenols include flavonoids (catechin, anthocyanin, flavone glycoside, isoflavone glycoside, flavane glycoside, flavanone, and rutin glycoside), phenolic acids (chlorogenic acid, ellagic acid, gallic acid, and propyl gallate), lignan glycosides, curcumin glycosides and coumarins. These compounds are contained in a large amount in an extract derived from natural substances. Therefore, the compounds can be used in the state of an extract.

From the viewpoint of accelerating a reduction reaction in the oil-water interface, the water-soluble antioxidant is an ascorbic acid compound.

As the water-soluble antioxidant, commercially available products may be appropriately used.

From the viewpoint of inhibiting the decomposition of the carotenoid and from the viewpoint of preservation stability, the content of the water-soluble antioxidant is preferably 0.001% by mass to 3.0% by mass, more preferably 0.01% by mass to 2.0% by mass, and even more preferably 0.05% by mass to 1.5% by mass, with respect to the total mass of the components contained in the water phase.

The content of the water-soluble antioxidant contained in the emulsion composition of the present invention is preferably 0.001% by mass to 3.0% by mass, more preferably 0.01% by mass to 2.0% by mass, and even more preferably 0.05% by mass to 1.5% by mass, with respect to the total mass of the emulsion composition.

<Other components>

The emulsion composition of the present invention may contain, as an oil phase component or a water phase component, other components as desired, in addition to the specific oil, the oil-soluble antioxidant, and the water-soluble antioxidant. Hereinafter, other components usable in the present invention will be described.

<(Poly)glycerin fatty acid ester>

Since the emulsion composition of the present invention contains a carotenoid as the oil-soluble antioxidant, it further contains (poly)glycerin fatty acid ester as an oil phase component contained in the emulsion particles.

A (poly)glycerin fatty acid ester not falling under the scope of the present invention has 1 to 5 glycerin units and 1 to 6 fatty acid units and has at least one hydroxyl group of the glycerin units.

The emulsion composition of the present invention contains a carotenoid as the oil-soluble antioxidant, and in a substance in which a predetermined (poly)glycerin fatty acid ester described above and a carotenoid are dissolved concurrently, the recrystallization of the carotenoid is inhibited.

The (poly)glycerin fatty acid ester, in which the number of glycerin units is equal to or less than 5, exhibits high affinity with carotenoid. In contrast, the (poly)glycerin fatty acid ester, in which the number of fatty acid units is equal to or less than 6, exhibits a strong effect of inhibiting crystallization of the carotenoid. Furthermore, if the emulsion composition contains the (poly)glycerin fatty acid ester containing a hydroxyl group of the glycerin units, the crystallization of the carotenoid can be sufficiently inhibited even when the carotenoid is used as the oil-soluble antioxidant.

From the viewpoint of inhibiting the recrystallization of the carotenoid, the (poly)glycerin fatty acid ester not falling under the scope of present claim 1 is preferably an ester of glycerin and a fatty acid, in which the number of the glycerin unit (average degree of polymerization) is 1 to 5 and more preferably 1 to 4, the number of the fatty acid unit is 1 to 6 and more preferably 1 to 5, and the fatty acid (for example, caprylic acid, capric acid, lauric acid, myristic acid, palmitic acid, stearic acid, oleic acid, linoleic acid, and behenic acid) has 8 to 22 carbon atoms and more preferably has 14 to 18 carbon atoms.

From the viewpoint of uniform solubility at the time of concurrent dissolution, the molecular weight of the aforementioned (poly)glycerin fatty acid ester is preferably equal to or less than 10,000, more preferably equal to or less than 3,000, and even more preferably equal to or less than 2,500.

In addition, from the viewpoint of the affinity with the carotenoid, the HLB of the (poly)glycerin fatty acid ester is preferably equal to or less than 9, and more preferably equal to or less than 6.

Examples of the (poly)glycerin fatty acid ester usable in the emulsion composition include glyceryl myristate, diglyceryl monostearate, triglyceryl monostearate, monoglyceric acid pentaglyceryl, triglyceryl dipalmitate, glyceryl distearate, tetraglyceryl tristearate and tetraglyceryl pentastearate. From the viewpoint of inhibiting recrystallization and from the viewpoint of uniform solubility, glyceryl myristate, glyceryl monostearate, diglyceryl monostearate, tetraglyceryl pentastearate, or tetraglyceryl tristearate is preferable.

From the viewpoint of the stability of the emulsion composition, the content (mass) of the (poly)glycerin fatty acid ester is preferably 0.01 times to 9 times the total mass of the carotenoid, more preferably 0.1 times to 8 times the total mass of the carotenoid, and even more preferably 0.3 times to 5 times the total mass of the carotenoid.

If the total mass of the polyglycerin fatty acid ester in the emulsion composition is 0.01 times the total mass of the carotenoid, the effect of sufficiently inhibiting the crystallization of the crystalline carotenoid can be expected. In contrast, if the total mass of the polyglycerin fatty acid ester is not more than 9 times the total mass of the carotenoid, the increase in the particle size of the emulsion particles can be inhibited when an emulsion is formed.

<Other oleaginous components>

The emulsion composition of the present invention may contain, as other components constituting the emulsion particles, other oleaginous components in addition to the respective components described above.

The aforementioned other oleaginous components are not particularly limited as long as they are components which dissolve in an amount of less than 0.3% by mass in water at 25°C and dissolve in an amount of equal to or greater than 5% by mass in the oil phase components in the present invention at 90°C. As the aforementioned other oleaginous components, the components having physical properties or functionality that suits the purpose can be appropriately selected and used. For example, fat-soluble vitamins (excluding vitamin E) are preferably used.

Examples of the fat-soluble vitamins include fatty acid esters of erythorbic acid such as erythorbyl palmitic acid ester and erythorbyl tetraisopalmitic acid ester; and fatty acid esters of vitamin $B_6$ such as pyridoxine dipalmitate, pyridoxine tripalmitate, pyridoxine dilaurate, and pyridoxine dioctanoate.

<Emulsifier>

The emulsion composition of the present invention may contain an emulsifier, which can be used as an oil phase component, in addition to the aforementioned components. Examples of such an emulsifier which can be used as an oil phase component include an emulsifier having HLB of equal to or less than 7 among the emulsifiers which will be described later.

The water phase in the emulsion composition of the present invention is preferably constituted with an aqueous solvent, particularly, water, and contains at least an emulsifier.

The emulsifier is a nonionic surfactant.

From the viewpoint of emulsifying power, the HBL of the emulsifier is equal to or greater than 10, and preferably equal to or greater than 12. If the HLB is too low, the emulsifying power becomes insufficient in some cases. From the viewpoint of a foam suppression effect, an emulsifier having HLB of equal to or greater than 5 and less than 10 may be concurrently used.

The HLB represents the balance between hydrophilicity and hydrophobicity that is generally used in the field of surfactants. For calculating the HLB, a generally used formula, for example, Kawakami's formula, can be used. Kawakami's formula is as below.

$$\mathrm{HLB} = 7 + 11.7 \log (M_w/M_0)$$

Herein, $M_w$ represents the molecular weight of a hydrophilic group, and $M_0$ represents the molecular weight of a hydrophobic group.

Furthermore, the HLB described in a catalog may also be used.

As is evident from the above formula, by utilizing the additivity of the HLB, an emulsifier having a certain HLB value can be obtained.

In preparing the emulsion composition of the present invention, from the viewpoint of obtaining fine emulsion particles and from the viewpoint of dispersion stability, the content of the emulsifier at the time of preparing emulsion particles having a particle size of equal to or less than 120 nm (preferably equal to or less than 100 nm) by performing an emulsification operation is preferably not less than 0.5 times the total mass of the oil phase components, more preferably not less than 0.55 times the total mass of the oil phase components, and even more preferably not less than 0.6 times the total mass of the oil phase components. The upper limit of the content of the emulsifier is not particularly limited. However, for example, the upper limit can be set to be not more than 3 times the total mass of the oil phase components.

From the viewpoint of inhibiting foaming of the emulsion composition, the content of the emulsifier is preferably equal to or less than 30% by mass, more preferably equal to or less than 20% by mass, and even more preferably equal to or less than 15% by mass, with respect to the total mass of the emulsion composition. The lower limit of the content of the emulsifier is not particularly limited. For example, the lower limit can be set to be equal to or greater than 1% by mass with respect to the total mass of the emulsion composition.

Among the emulsifiers, a nonionic surfactant is used because it is less irritating and exerts a small influence on the environment. From the viewpoint of obtaining fine emulsion particles, the polyglycerin fatty acid ester used according to the present invention is particularly preferable.

Therefore, the emulsion composition contains an emulsifier having HLB of equal to or greater than 10. As the emulsifier having HLB of equal to or greater than 10, polyglycerin fatty acid ester is used.

In one of the preferred embodiments of the emulsion composition of the present invention, the emulsion composition contains the emulsifier having HLB of equal to or greater than 10 in such an amount that is equal to or greater than 50% by mass of the amount of the oil phase and is equal to or less than 15% by mass with respect to the total mass of the emulsion composition. As the emulsifier having HLB of equal to or greater than 10, polyglycerin fatty acid ester is used.

Regarding the sucrose fatty acid ester (not falling under the scope of the present invention), from the viewpoint of the stability of the dispersed particles in the composition, the number of carbon atoms of the fatty acid constituting the sucrose fatty acid ester is preferably 12 to 20 and more preferably 14 to 16.

Preferred examples of the sucrose fatty acid ester include sucrose dioleic acid ester, sucrose distearic acid ester, sucrose dipalmitic acid ester, sucrose dimyristic acid ester, sucrose dilauric acid ester, sucrose monooleic acid ester, sucrose monostearic acid ester, sucrose monopalmitic acid ester, sucrose monomyristic acid ester and sucrose monolauric acid ester. Among these, sucrose monooleic acid ester, sucrose monostearic acid ester, sucrose monopalmitic acid ester, sucrose monomyristic acid ester, and sucrose monolauric acid ester are more preferable.

These sucrose fatty acid esters can be used singly or used by being mixed with each other.

The water phase composition contains polyglycerin fatty acid ester as an emulsifier.

The polyglycerin fatty acid ester is an ester of polyglycerin and a fatty acid, in which an average degree of polymerization of the polyglycerin is equal to or greater than 6, preferably 6 to 15, and more preferably 8 to 10, and the fatty acid has 8 to 18 carbon atoms. Examples of the fatty acid include caprylic acid, capric acid, lauric acid, myristic acid, palmitic acid, stearic acid, oleic acid, and linoleic acid.

Preferred examples of the polyglycerin fatty acid ester include hexaglycerin monooleic acid ester, hexaglycerin monostearic acid ester, hexaglycerin monopalmitic acid ester, hexaglycerin monomyristic acid ester, hexaglycerin monolauric acid ester. The polyglycerin fatty acid ester used according to the present invention is at least one compound selected from the group consisting of decaglycerin monooleic acid ester, decaglycerin monostearic acid ester, decaglycerin monopalmitic acid ester, decaglycerin monomyristic acid ester and decaglycerin monolauric acid ester.

Among these, decaglycerin monooleic acid ester (HBL = 12), decaglycerin monostearic acid ester (HLB = 12), decaglycerin monopalmitic acid ester (HLB = 13), decaglycerin monomyristic acid ester (HLB 14) and decaglycerin monolauric acid ester (HLB = 16), are more preferable.

In the present invention, from the viewpoint of the stability of the oil-soluble antioxidant, the number of carbon atoms of the fatty acid group of the polyglycerin fatty acid ester, which is used as an emulsifier and has HLB of equal to or greater than 10, is preferably equal to or less than 16 (more preferably 15 and even more preferably 14). It is considered that if polyglycerin fatty acid ester containing a fatty acid group having less carbon atoms is used as the polyglycerin fatty acid ester having HLB of equal to or greater than 10, the reaction between the oil-soluble antioxidant and the water-soluble antioxidant caused in the oil-water interface may be accelerated.

In the present invention, as an emulsifier, these polyglycerin fatty acid esters can be used singly or used by being mixed with each other.

In the present invention, the number of carbon atoms of the fatty acid of the sorbitan fatty acid ester is preferably equal to or greater than 8 and more preferably equal to or greater than 12. Preferred examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate and sorbitan trioleate.

In the present invention, these sorbitan fatty acid esters can be used singly or used by being mixed with each other.

The number of carbon atoms of the fatty acid of the polyoxyethylene sorbitan fatty acid ester is preferably equal to or greater than 8 and more preferably equal to or greater than 12. Furthermore, the length of ethylene oxide (number of moles of ethylene oxide added) of polyoxyethylene is preferably 2 to 100 and more preferably 4 to 50.

Preferred examples of the polyoxyethylene sorbitan fatty acid ester include polyoxyethylene sorbitan monocaprylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate and polyoxyethylene sorbitan trioleate.

These polyoxyethylene sorbitan fatty acid esters can be used singly or used by being mixed with each other.

An emulsion composition not falling under the scope of the present invention may contain, as the emulsifier in the present invention, phospholipid such as lecithin.

The phospholipid is a compound in which a base and a polyol, are bonded to essential constituents such as a glycerin skeleton, a fatty acid residue, and a phosphoric acid residue. The phospholipid is also called lecithin. Having a hydrophilic group and a hydrophobic group in a molecule, the phospholipid has been conventionally widely used as an emulsifier in the field of foods, medicines, and cosmetics.

Industrially, lecithin having purity of equal to or higher than 60% is used, and such a lecithin can also be used in the present invention. However, from the viewpoint of forming oil droplets having a small particle size and from the viewpoint of the stability of the functional oleaginous components, it is preferable to use lecithin which is generally called high-purity lecithin. The purity of such a lecithin is equal to or higher than 80% and preferably equal to or higher than 90%.

Examples of the phospholipid include various conventionally known phospholipids that are extracted and separated from the living body of plants, animals, and microorganisms.

Specific examples of such phospholipids include various lecithins derived from plants such as soybeans, corn, peanuts, rape seeds, and barley, egg yolk, animals such as cows, microorganisms such as E. coli .

Examples of the compounds names of the lecithin include phosphatidic acid; glycerolecithin such as phosphatidylglycerin, phosphatidylinositol, phosphatidylethanolamine, phosphatidyl methylethanolamine, phosphatidylcholine, phosphatidylserine, bisphosphatidic acid, and diphosphatidylglecerin (cardiolipin); sphingolecithin such as sphingomyelin.

Furthermore, in addition to the aforementioned high-purity lecithin, hydrogenated lecithin, enzymatically decomposed lecithin, enzymatically decomposed hydrogenated lecithin and hydroxylecithin, can be used. These lecithins can be used singly or used in the form of a mixture of plural kinds of thereof.

<Polyol>

For the purpose of obtaining antiseptic properties and regulating viscosity and particle size, the emulsion composition of the present invention may contain a polyol. One kind of the polyol may be used singly, or two or more kinds thereof may be used concurrently.

The polyol is not particularly limited as long as it is an alcohol having a valency of equal to or higher than 2. Examples of the polyol include glycerin, diglycerin, triglycerin, polyglycerin, 3-methyl-1,3-butanediol, 1,3-butylene glycol, isoprene glycol, polyethylene glycol, 1,2-pentanediol, 1,2-hexanediol, propylene glycol, dipropylene glycol, polypropylene glycol, ethylene glycol, diethylene glycol, pentaerythritol, neopentyl glycol, maltitol, reduced sugar syrup, sucrose, lactitol, palatinit, erythritol, sorbitol, mannitol, xylitol, xylose, glucose, lactose, mannose, maltose, galactose, fructose, inositol, pentaerythritol, maltotriose, sorbitan, trehalose, sugar obtained by the decomposition of starch, an alcohol prepared by the reduction of the sugar obtained by the decomposition of starch.

The polyol can be contained in the emulsion composition of the present invention at any ratio with respect to the total mass of the water phase composition.

<Other components to be added>

In addition to the respective components described above, components that are generally used in the field of foods, cosmetics, and the like may be appropriately mixed with the emulsion composition of the present invention according to the form of the composition. Depending on their characteristics, the components to be added may be mixed with the emulsion composition as components of the oil phase composition or components of the water phase composition, or may be mixed with the emulsion composition as components to be added to the water phase of the emulsion composition.

Examples of other components described above include monosaccharide or polysaccharide such as glucose, fructose, lactose, maltose, sucrose, pectin, kappa carrageenan, locust bean gum, guar gum, hydroxypropyl guar gum, xanthan gum, karaya gum, tamarind seed polysaccharide, gum Arabic, gum tragacanth, hyaluronic acid, sodium hyaluronate, sodium chondroitin sulfate, and dextrin; sugar alcohols such as sorbitol, mannitol, maltitol, lactose, maltotriitol, and xylitol; inorganic salts such as sodium chloride and sodium sulfate; proteins having a molecular weight of greater than 5,000, such as casein, albumin, methylated collagen, hydrolyzed collagen, water-soluble collagen, and gelatin; synthetic polymers such as a carboxyvinyl polymer, sodium polyacrylate, polyvinyl alcohol, polyethylene glycol, and an ethylene oxide·propylene oxide block copolymer; water-soluble cellulose derivatives such as hydroxyethyl cellulose·methyl cellulose. Based on their function, each of these components may be contained as, for example, a functional component, an excipient, a viscosity adjuster, or the like in the emulsion composition of the present invention.

Furthermore, for example, other additives that are generally used for the purpose of their own, such as various components having medicinal properties, a pH adjuster, a pH buffer, an ultraviolet absorber, a preservative, fragrance, and a colorant, can be concurrently used.

<pH>

In view of emulsification stability, the pH of the emulsion composition of the present invention is preferably 4.0 to 10.0, and more preferably 5.0 to 9.0.

<Size of emulsion particles>

From the viewpoint of accelerating the reduction of the oil-soluble antioxidant by the water-soluble antioxidant and from the viewpoint of the permeability of the emulsion particles into the skin of a living body, the average particle size of the emulsion particles contained in the emulsion composition of the present invention is equal to or less than 120 nm, more preferably equal to or less than 100 nm, and even more preferably equal to or less than 80 nm. The lower limit of the average particle size of the emulsion particles is not particularly limited and can be set to be, for example, equal to or greater than 0.8 nm.

In the present invention, a range of the average particle size of the emulsion particles can be set by combining the upper limit and the lower limit described later. For example, the range of the average particle size can be set to be equal to or greater than 0.8 nm and equal to or less than 120 nm.

In the present invention, the average particle size of the emulsion particles means the volume average particle size of the emulsion particles present in the emulsion composition. The average particle size of the emulsion particles contained in the emulsion composition of the present invention is measured by using a dynamic light scattering method because the method makes it possible to accurately measure the average particle size in a simple way.

Examples of commercially available measurement apparatuses using the dynamic light scattering method include a concentrated system particle size analyzer FPAR-1000 (OTSUKA ELECTRONICS CO., LTD.), Nanotrac UPA (NIKKISO CO., LTD.), Nanosizer (manufactured by Malvern Instruments, Ltd.). In the present invention, as the average particle size of the emulsion particles, a value measured at 23°C by using the concentrated system particle size analyzer FPAR-1000 (OTSUKA ELECTRONICS CO., LTD.) is adopted. The particle size is measured by a method in which a sample collected from the emulsion composition of the present invention is diluted with pure water such that the concentration of oil components contained in the sample becomes 1% by mass, and then the particle size is measured by using a quartz cell. For measuring the volume average particle size, a refractive index of the sample is set to be 1.600, a refractive index of a dispersion medium is set to be 1.333 (pure water), and the viscosity of pure water is regarded as the viscosity of the dispersion medium. A volume average particle size measured under these conditions can be determined to be the particle size.

Specifically, the emulsion composition as the sample to be measured is diluted with Milli-Q water by a factor of 10 so as to measure the particle size, and a median diameter (d = 50) obtained as a result is determined to be the particle size.

The average particle size of the emulsion particles can be adjusted by the factors such as stirring conditions (shear force, temperature, or pressure) in a manufacturing method of the emulsion composition and a ratio between the oil phase and the water phase, in addition to the components of the composition.

<Manufacturing method of emulsion composition>

The emulsion composition of the present invention is preferably manufactured by a manufacturing method including at least mixing the oil phase composition, which contains the specific oil and the oil-soluble antioxidant, with the water phase composition.

In the manufacturing method of the emulsion composition of the present invention, the addition of the water-soluble antioxidant to the water phase may be performed by any of an embodiment in which the water-soluble antioxidant is added to the water phase composition that has not yet been mixed with the oil phase composition and an embodiment in which the water-soluble antioxidant is added to the emulsion composition that is prepared by mixing the oil phase composition with the water phase composition. However, it is more preferable to add the water-soluble antioxidant to the water phase by the latter embodiment.

The water-soluble antioxidant may be directly added to the water phase composition or to the prepared emulsion composition. However, it is more preferable to prepare an aqueous solution containing the water-soluble antioxidant and then add the aqueous solution to the water phase composition or to the prepared emulsion composition. The aqueous solution may contain any types of additives as desired.

Examples of a preferred manufacturing method of the emulsion composition of the present invention include a method in which the oil-in-water emulsion composition, which contains the specific oil and the oil-soluble antioxidant in the oil phase, is prepared in advance, and then the aqueous solution containing the water-soluble antioxidant that is used as a diluent is added to the obtained oil-in-water emulsion composition.

In the present invention, one of the preferred embodiments for obtaining the oil phase composition containing the specific oil and carotenoid as the oil-soluble antioxidant is an embodiment including heating the oil phase composition, which has not yet been mixed with the water phase composition as will be described later, under a temperature condition of equal to or higher than 90°C. In a case in which the oil phase composition is obtained by such an embodiment, considering the use of the thermally decomposed water-soluble antioxidant such as an ascorbic acid compound, an embodiment is preferable in which the emulsion composition containing the specific oil and carotenoid as the oil-soluble antioxidant is prepared, and then the water-soluble antioxidant is added to the emulsion composition.

Hereinafter, the preferred manufacturing method of the emulsion composition of the present invention will be more specifically described by illustrating a method of using carotenoid as the oil-soluble antioxidant.

As one of the preferred embodiments for obtaining the oil phase composition containing the specific oil and carotenoid as the oil-soluble antioxidant, an embodiment is exemplified which includes heating a liquid of oil phase component mixture, which contains the specific oil, a carotenoid, (poly)glycerin fatty acid ester having 1 to 3 glycerin units, 1 to 6 fatty acid units, and at least one hydroxyl group of the glycerin units, and the antioxidant, under a temperature condition of equal to or higher than 90°C.

Hereinafter, by illustrating the manufacturing method of the emulsion composition using the oil phase composition containing the specific oil and a carotenoid as the oil-soluble antioxidant, the manufacturing method of the emulsion composition will be specifically described. However, the present invention is not limited thereto.

According to the manufacturing method of the present embodiment, a carotenoid is heated together with a predetermined (poly)glycerin fatty acid ester and an antioxidant, under a condition of a temperature of equal to or higher than the dissolution temperature of the carotenoid. Therefore, the carotenoid dissolves concurrently with the predetermined (poly)glycerin fatty acid ester. If a carotenoid-containing oil phase composition obtained by the concurrent dissolution is used as the oil phase composition which is heated and emulsified together with the water phase composition, an emulsion composition obtained by emulsification becomes a composition in which the crystallization of crystalline carotenoid such as lycopene is inhibited.

In the manufacturing method described herein, first, a specific oil, a carotenoid, (poly)glycerin fatty acid ester, which has 1 to 6 glycerin units, 1 to 6 fatty acid units, and at least one hydroxyl group of the glycerin units, and an antioxidant are mixed together, and in this way, a liquid of an oil phase component mixture can be obtained (hereinafter, referred to as a "step of mixing oil phase components"). If necessary, the liquid of an oil phase component mixture may contain other oil phase components.

Specific examples of the specific oil, a carotenoid, (poly)glycerin fatty acid ester, antioxidant, and other components contained in the liquid of an oil phase component mixture used in the step of mixing oil phase components, the content of each of the above components, and a preferred range of the content are the same as those described above for each of the components contained in the emulsion composition of the present invention.

Thereafter, by heating the liquid of an oil phase component mixture under a temperature condition of equal to or higher than 90°C, an oil phase composition can be obtained (hereinafter, referred to as a "step of heating oil phase components"). The heating temperature just needs to be equal to or higher than 90°C, and can be set to be 90°C to 155°C. From the viewpoint of inhibiting thermal decomposition, the heating temperature is preferably 110°C to 150°C, and more preferably 120°C to 145°C.

The heating time in the step of heating oil phase components just needs to be set such that the carotenoid in the liquid of an oil phase component mixture dissolves. From the viewpoint of efficiently making the crystals amorphous and from the viewpoint of inhibiting the carotenoid from being decomposed due to excessive heat, the heating time is preferably 10 minutes to 60 minutes, and more preferably 5 minutes to 45 minutes. However, the present invention is not limited thereto.

By the aforementioned heating treatment, it is possible to obtain an oil phase composition from the liquid of an oil phase component mixture containing carotenoid.

In the heating treatment, it is preferable to make the entirety of the liquid of an oil phase component mixture have a uniform temperature. Accordingly, it is preferable to thoroughly stir the liquid of an oil phase component mixture while heating it. Furthermore, it is desirable to hold the liquid of an oil phase component mixture at a constant temperature while stirring and heating the liquid by using an airtight container.

By the step of heating oil phase components, an oil phase composition is obtained.

The manufacturing method of the present embodiment includes, after the step of heating oil phase components, emulsifying the oil phase composition obtained by the step of heating oil phase components and a water phase composition containing a predetermined water phase component (the water phase composition may contain a water-soluble antioxidant) (step of emulsification). By the step of emulsification, it is possible to obtain an oil-in-water emulsion composition in which the oil phase components containing carotenoid are dispersed as fine oil droplets (emulsion particles) in water. In this composition, carotenoid remains stable.

In the heating treatment, it is preferable to make the entirety of the liquid of an oil phase component mixture have a uniform temperature. Accordingly, it is preferable to thoroughly stir the liquid of an oil phase component mixture while heating it. Furthermore, it is desirable to hold the liquid of an oil phase component mixture at a constant temperature while stirring and heating the liquid by using an airtight container.

By the step of heating oil phase components, an oil phase composition is obtained.

The manufacturing method of the present embodiment includes, after the step of heating oil phase components, emulsifying the oil phase composition obtained by the step of heating oil phase components and a water phase composition containing a predetermined water phase component (the water phase composition may contain a water-soluble antioxidant) (step of emulsification). By the step of emulsification, it is possible to obtain an oil-in-water emulsion composition in which the oil phase components containing the specific oil and a carotenoid are dispersed as fine oil droplets (emulsion particles) in water. In this composition, the carotenoid remains stable.

The ratio (mass) of the oil phase to the water phase during the emulsification and dispersion is not particularly limited. However, the ratio of oil phase/water phase (% by mass) is preferably 0.1/99.9 to 50/50, more preferably 0.5/99.5 to 30/70, and even more preferably 1/99 to 20/80.

It is preferable for the ratio of oil phase/water phase to be within the above range, because an emulsion composition is obtained which sufficiently contains active components and has stability sufficient for practical use.

During pressure emulsification, an emulsification operation may be performed by a single step. However, from the viewpoint of obtaining uniform and fine emulsion particles (emulsified particles), it is preferable to perform the emulsification operation by two or more steps.

Specifically, it is particularly preferable to use two or more kinds of emulsification apparatuses in a method in which emulsification is performed by using a high-pressure homogenizer in addition to an emulsification operation performed by a single step of performing emulsification by using a general emulsification apparatus (for example, a stirrer, an impeller stirrer, a homomixer, or a continuous circulation-type shearing apparatus) that utilizes shearing action. If the high-pressure homogenizer is used, the emulsion particles in the emulsion composition can be more uniform and fine droplets. For the purpose of obtaining droplets having a more uniform particle size, the emulsification operation may be performed plural times.

As means for emulsification that can be used herein, it is possible to use any of generally known emulsification methods such as a spontaneous emulsification method, an emulsification method based on surface chemistry, an electrical emulsification method, a capillary emulsification method, a mechanical emulsification method, and an ultrasonic emulsification method.

As a method that is useful for obtaining finer emulsion particles in the emulsion composition, an emulsification method based on surface chemistry such as a PIT emulsification method, or a gel emulsification method is known. The method has an advantage that it consumes a small amount of energy, and accordingly, the method is suitable for finely emulsifying materials which easily deteriorate by heat.

As an emulsification method used for various purposes, a method using a mechanical force, that is, a method of dividing oil droplets by applying a strong shear force from the outside, is used. Among apparatuses using a mechanical force, a high-speed and high-shear stirrer is most commonly used. As such a stirrer, those called a homomixer, a disper mixer, and an ultra mixer are commercially available.

As another mechanical emulsification apparatus useful for obtaining fine particles, there is a high-pressure homogenizer, and various high-pressure homogenizers are commercially available. The high-pressure homogenizer makes it possible to apply a stronger shear force compared to a stirring method, and accordingly, it is possible to obtain fine particles even when the amount of the emulsifier is relatively small.

The high-pressure homogenizer is roughly classified into a chamber type high-pressure homogenizer which has a fixed throttle portion and a homogenous valve type high-pressure homogenizer which controls the aperture of a throttle.

Examples of the chamber type high-pressure homogenizer include Microfludizer (manufactured by Microfluidics), Nanomizer (manufactured by Yoshida Kikai Co., Ltd.), Ultimaizer (manufactured by SUGINO MACHINE LIMITED).

Examples of the homogenous valve type high-pressure homogenizer include a Gaulin type homogenizer (manufactured by APV), a Lannier type homogenizer (manufactured by Lannier, Inc.), a high-pressure homogenizer (manufactured by Niro Soavi), a homogenizer (manufactured by SANWA ENGINEERING, LTD.), a high-pressure homogenizer (manufactured by IZUMI FOOD MACHINERY CO., LTD.), an ultrahigh-pressure homogenizer (manufactured by IKA).

As an emulsification apparatus which is a dispersion apparatus showing relatively excellent energy efficiency and having a simple structure, there is an ultrasonic homogenizer. Examples of a high-power ultrasonic homogenizer that can also be used for manufacturing include ultrasonic homogenizers US-600, US-1200T, RUS-1200T, and MUS-1200T (manufactured by NISSEI Corporation), ultrasonic processors UIP 2000, UIP-4000, UIP-8000, and UIP-16000 (manufactured by Hielscher Ultrasonics Gmbh). These high-power ultrasonic irradiation apparatuses are used at a frequency of equal to or less than 25 kHz, and preferably at a frequency of 15 kHz to 20 kHz.

Furthermore, a method of using a static mixer, a microchannel, a micromixer, a membrane emulsification apparatus, which does not have an external stirring portion and consumes only a small amount of energy, is also useful as another known means for emulsification.

In the manufacturing method of the present embodiment, the temperature condition at the time of emulsification and dispersion is not particularly limited. However, from the viewpoint of the stability of a functional oleaginous component such as a carotenoid, the temperature is preferably 10°C to 100°C. According to the melting point of the functional oleaginous component to be handled, the temperature condition within a preferred range can be appropriately selected.

When a high-pressure homogenizer is used in the present invention, the pressure thereof is preferably equal to or greater than 50 MPa, more preferably 50 MPa to 280 MPa, and even more preferably 100 MPa to 280 MPa.

From the viewpoint of maintaining the particle size of the emulsion particles, it is preferable that the emulsified and dispersed composition is cooled through any type of cooler within 30 seconds and preferably within 3 seconds immediately after passing through the chamber.

In the manufacturing method of the present embodiment, when an embodiment, in which the water-soluble antioxidant is added to the prepared emulsion composition, is adopted as an embodiment for adding the water-soluble antioxidant to the water phase, an aqueous solution containing the water-soluble antioxidant may be added to the emulsion composition obtained as described above.

<Use>

In the emulsion composition of the present invention, the decomposition of the lycopene as the oil-soluble antioxidant is inhibited. Furthermore, the emulsion composition of the present invention is excellent in preservation stability. Therefore, the emulsion composition of the present invention is expected to sufficiently bring about intended effects. Consequently, the emulsion composition of the present invention can be preferably used in a food composition, a cosmetic composition and a medicinal composition.

If necessary, the components that can be added to foods or external preparations for skin such as cosmetics can be added to foods or external preparations for skin such as cosmetics that contain the emulsion composition of the present invention.

The foods and cosmetics containing the emulsion composition of the present invention can exhibit the effect, for example, excellent preservation stability that is not sufficiently exhibited in some cases due to the decomposition of the oil-soluble antioxidant and the deterioration of the emulsion state.

The emulsion composition of the present invention is preferably used in cosmetics such as a toner, a serum, an emulsion, a cream-type facial pack·mask, a facial pack, cosmetics for shampooing, cosmetics for fragrance, a liquid body cleanser, cosmetics for UV care, cosmetics for deodorant, and cosmetics for oral care.

Furthermore, the emulsion composition of the present invention is preferably used not only in general foods such as a nutritional drink, an analeptic, a favorite drink, and a frozen dessert but also in capsule-type nutritional food supplements.

Examples

Hereinafter, the present invention will be described based on examples, but the present invention is not limited thereto. In the following description, unless otherwise specified, the values represented by "part" or "%" are based on mass.

[Examples 1 to 14 and Comparative examples 1 to 10]

Each of the emulsion compositions of Examples 1 to 14 (including Reference Examples 2 to 6 and 11 to 14) and Comparative examples 1 to 10 was prepared as below.

1. Preparation of emulsions A to S (Preparations A to E, H to L and P to T do not fall under the scope of the present invention but are provided to better understand the present invention

<Preparation of emulsion A> (for reference)

The following oil composition was heated and mixed for 5 minutes on a hot plate at 135°C while being stirred, and whether the composition was mixed well was checked.

The following water phase composition was heated and mixed in a constant-temperature tank at 70°C while being stirred, and whether the composition was mixed well was checked. The composition was hold at 70°C.

The water phase composition was added to the oil phase composition, and the resultant was stirred, mixed, and dispersed by using an ultrasonic homogenizer, thereby obtaining a crude composition. Thereafter, by using a high-pressure emulsification apparatus (Ultimaizer, manufactured by SUGINO MACHINE LIMITED), the obtained crude composition was emulsified at a high pressure under emulsification conditions of an emulsification pressure of 245 MPa and a pass number (number of times the emulsification operation was performed) of 3.

In this way, an emulsion A was obtained.

|  -Oil phase composition- | |
| --- | --- |
| (1) Lyc-O-Mato 80% (extract derived from tomato, lycopene content: 80%) *1 | 0.21 parts |
| (2) Glycerin tri(caprylate·caprate) *2 | 13.87 parts |
| (3) Mixed tocopherol *3 | 0.64 parts |
| (4) Diglyceryl monostearate *4 | 0.28 parts |

*1: "Lyc-O-Mato 80%" manufactured by LYCORED, LTD., oil-soluble antioxidant
*2: "COCONAD MT" ($C_8$/$C_{10}$ triglyceride, HLB = 1, I/O value: 0.27 to 0.33) manufactured by Kao Corporation.
*3: "Riken E oil 800" manufactured by RIKEN VITAMIN Co., Ltd., oil-soluble antioxidant
*4: "NIKKOL DGMS" (HLB = 5.0) manufactured by Nikko Chemicals Co., Ltd.

|  -Water phase composition- | |
| --- | --- |
| (1) Decaglyceryl myristate *5 | 3 parts |
| (2) Glycerin | 45.0 parts |
| (3) Purified water | 30.0 parts |

*5: "Decaglyn-1M" (HLB = 14.0) manufactured by Nikko Chemicals Co., Ltd.

<Preparation of emulsions B to N> (emulsions B to E, H to L for reference)

Emulsions B to N were obtained in the same manner as the manner used for obtaining the emulsion A, except that, in Preparation of emulsion A, the type and content of each of the components used for preparing the oil phase composition and the water phase composition were changed as shown in Table 2.

<Preparation of emulsion O> (for reference)

An emulsion O was obtained in the same manner as the manner used for obtaining the emulsion A, except that, in Preparation of emulsion A, the type and content of each of the components used for preparing the oil phase composition and the water phase composition were changed as shown in Table 2, and the temperature of the hot plate at the time of preparing the oil phase composition was changed to 50°C.

<Preparation of emulsions P to T> (for reference)

Emulsions P to T were obtained in the same manner as the manner used for obtaining the emulsion A, except that, in Preparation of emulsion A, the type and content of each of the components used for preparing the oil phase composition and the water phase composition were changed as shown in Table 2, and the temperature of the hot plate at the time of preparing the oil phase composition was changed to 70°C.

In Table 2, the numerical value showing the amount of each of the components mixed represents "part by mass".

Specifically, among the components described in Table 2, the components other than the aforementioned components *1 to *5 are as follows.

- Hematococcus algae extract (containing 20% of astaxanthin): "ASTOTS-S" manufactured by Takedashiki Co., Ltd., oil-soluble antioxidant
- Tocotrienol-containing substance (containing 67% of tocotrienol): "Tocotrit 92" manufactured by Eisai Food & Chemical Co., Ltd., oil-soluble antioxidant
- Diethylhexyl sebacate: "Neosolue-EHS" manufactured by Nippon Fine Chemical
- Isopropyl myristate: "NIKKOL IPM-EX" manufactured by Nikko Chemicals Co., Ltd.
- Isopropyl isostearate: "NIKKOL IPIS" manufactured by Nikko Chemicals Co., Ltd.
- Dialkyl carbonate (C14, 15): "Lialcarb SR-1000/R" manufactured by Nikko Chemicals Co., Ltd.
- Cetyl ethylhexanoate: "Lialcarb SR-1000/R" manufactured by Nikko Chemicals Co., Ltd.
- Isostearyl isostearate: "ISIS" manufactured by KOKYU ALCOHOL KOGYO CO., LTD.
- Liquid paraffin: "Hicol K-230" manufactured by KANEDA Co., Ltd.
- Octyldodecyl myristate: "NIKKOL ODM-100" manufactured by Nikko Chemicals Co., Ltd.
- Decaglyceryl oleate: "Decaglyn 1-O" (HLB = 12.8) manufactured by Nikko Chemicals Co., Ltd.
- Glycerin: "Concentrated glycerin for cosmetics" manufactured by Kao Corporation
- Sucrose stearic acid ester: "RYOTO sugar ester S-1670" manufactured by Mitsuibishi-kagaku Foods Corporation
- Lecithin: "Reshion P" manufactured by RIKEN VITAMIN Co., Ltd.

[Table 2]

| | | A | B | C | D | E | F | G | H | I | J | K | L | M | N | O | P | Q | R | S | T |
|---|---|---|---|---|---|---|---|---|---|---|---|---|---|---|---|---|---|---|---|---|---|
| Oil phase | Lyc-O-Mato 80% (tomato extract containing 80% of lycopene) | 0.21 | 0.21 | 0.21 | 0.21 | 0.21 | 0.21 | 0.21 | 0.21 | 0.21 | 0.21 | 0.21 | 0.21 | 0.21 | 0.21 | 0.21 | | | | | |
| | ASTOTS-S (hematococcus algae extract containing 20% of astaxanthin) | | | | | | | | | | | | | | | | 1.00 | 1.00 | | | 1.50 |
| | Tocotri 92 (containing 67% of tocotrienol) | | | | | | | | | | | | | | | | | | 4.00 | 4.00 | 9.30 |
| | Glycerin tri(caprylate·caprate) (I/O value: 0.27 to 0.33) | 13.87 | 13.87 | 13.87 | | | | | | | | | | | | | 13.36 | | 11.00 | | |
| | Diethylhexyl sebacate (I/O value: 0.24) | | | | 13.87 | | | | | | | | | | | | | | | | |
| | Isopropyl myristate (I/O value: 0.18) | | | | | 13.87 | | | | | | | | | | | | | | | |
| | Isopropyl isostearate (I/O value: 0.15) | | | | | | | | 13.87 | | | | | | | | | | | | |
| | Dialkyl carbonate (C14, 15) (I/O value: 0.13) | | | | | | | | | 13.87 | | | | | | | | | | | |
| | Cetyl ethylhexanoate (I/O value: 0.13) | | | | | | | | | | 13.87 | | | | | | | | | | |
| | Isostearyl isostearate (I/O value: 0.09) | | | | | | | | | | | 13.87 | | | | | | | | | |
| | Liquid paraffin (I/O value: 0) | | | | | | | | | | | | 13.87 | | | | | | | | |
| | Octyldodecyl myristate (I/O value: 0.09) | | | | | | 13.87 | 13.87 | | | | | | 13.87 | 13.87 | 13.87 | | 13.36 | | 11.00 | |
| | Mixed tocopherol | 0.64 | 0.64 | 0.64 | 0.64 | 0.64 | 0.64 | 0.64 | 0.64 | 0.64 | 0.64 | 0.64 | 0.64 | 0.64 | 0.64 | 0.64 | 0.64 | 0.64 | | | 3.20 |
| | Lecithin | | | | | | | | | | | | | | | | | | | | 1.00 |
| | Diglyceryl monostearate | 0.28 | 0.28 | 0.28 | 0.28 | 0.28 | 0.28 | 0.28 | 0.28 | 0.28 | 0.28 | 0.28 | 0.28 | 0.28 | 0.28 | 0.28 | | | | | |
| | Decaglyceryl oleate | | 10.0 | | | | | | | | | | | | 10.0 | | | | | | 6.7 |
| | Decaglyceryl myristate | 3.0 | | 10.0 | 10.0 | 10.0 | 3.0 | 5.0 | 10.0 | 10.0 | 10.0 | 10.0 | 10.0 | 10.0 | | 10.0 | 10.0 | 10.0 | 10.0 | 10.0 | |
| Water phase | Sucrose stearic acid ester | | | | | | | | | | | | | | | | | | | | 3.3 |
| | Glycerin | 45.0 | 45.0 | 45.0 | 45.0 | 45.0 | 45.0 | 45.0 | 45.0 | 45.0 | 45.0 | 45.0 | 45.0 | 45.0 | 45.0 | 45.0 | 45.0 | 45.0 | 45.0 | 45.0 | 45.0 |
| | Water | 30.0 | 30.0 | 30.0 | 30.0 | 30.0 | 30.0 | 30.0 | 30.0 | 30.0 | 30.0 | 30.0 | 30.0 | 30.0 | 30.0 | 30.0 | 30.0 | 30.0 | 30.0 | 30.0 | 30.0 |
| | Particle size immediately after preparation | 160 nm | 67 nm | 66 nm | 71 nm | 62 nm | 162 nm | 115 nm | 67 nm | 65 nm | 65 nm | 61 nm | 62 nm | 58 nm | 60 nm | 115 nm | 70 nm | 69 nm | 66 nm | 64 nm | 58 nm |
| | Presence or absence of crystals (polarized light microscopy) | Absent | Absent | Absent | Absent | Absent | Absent | Absent | Absent | Absent | Absent | Absent | Absent | Absent | Absent | Present | Absent | Absent | Absent | Absent | Absent |

18

In Table 2 emulsions A to E, H to L and P to T do not fall under the scope of the present invention

For the emulsions A to T obtained as above, the particle size immediately after preparation and the absence or presence of crystals were checked in the following manner. The results are also shown in Table 2.

-Particle size-

5 ml glass vials were filled with each of the emulsions A to T, thereby preparing samples. Purified water was added to each of the samples so as to prepare 0.33% by mass of a diluted solution, and the volume-based average particle size (median particle size) of the particles in the diluted solution was measured by using a dynamic light scattering analyzer (trade name: FPAR-1000, manufactured by OTSUKA ELECTRONICS CO., LTD.).

-Presence or absence of crystals-

By using a polarized light microscope (trade name: PCLIPSE LV100POL, manufactured by Nikon Corporation), each of the emulsion A to T was visually observed, and based on the following criteria, the presence or absence of crystals in the emulsion was checked. The observation criteria are as follows.

Absent: crystals are not observed.
Present: crystals are observed.

2. Preparation of emulsion composition

Each of the emulsions A to S obtained as above was mixed with a diluted solution (aqueous solution containing a water-soluble antioxidant) having the composition shown in the following Table 4 while being stirred at 25°C such that the composition described in Table 4 was obtained. In this way, each of the emulsion compositions of Examples 1 to 14 (including Reference Examples 2-6 and 11 to 14) and Comparative examples 1 to 10 was prepared.
As the components in Table 4, the following components were used.

• Ascorbic acid: a reagent "L(+)-ascorbic acid" manufactured by Wako Pure Chemical Industries, Ltd.
• Gallic acid: "gallic acid" manufactured by Wako Pure Chemical Industries, Ltd.
• Hydroquinone: "hydroquinone" manufactured by Wako Pure Chemical Industries, Ltd.
• Ascorbyl Mg phosphate: "L-ascorbyl magnesium phosphate" manufactured by Wako Pure Chemical Industries, Ltd.
• Citric acid: "citric acid" manufactured by Wako Pure Chemical Industries, Ltd.

In Table 3, the numerical value showing the amount of each of the components mixed represents "part by mass".

3. Evaluation

For each of the emulsion compositions of examples and comparative examples obtained as above, as an index of preservation stability, a lycopene decomposition rate obtained after the emulsion compositions were preserved for 4 weeks at 50°C was evaluated. The evaluation results are also shown in Table 4.

<Evaluation of decomposition rate of oil-soluble antioxidant>

Each of the emulsion compositions of examples and comparative examples obtained as above was divided into two fractions, and 5 ml glass vials were filled with the divided composition. One of the glass vials was preserved for 4 weeks at 50°C, thereby preparing a sample for evaluation. Among the components which were contained as the oil-soluble antioxidant in each of the just prepared sample and the sample preserved for 4 weeks, for lycopene and astaxanthin, the decomposition rate (%) was calculated as below based on a measurement value obtained by measuring absorbance, and for tocotrienol, the decomposition rate (%) was calculated as below based on a measurement value obtained by high performance liquid chromatography (HPLC). The lower the decomposition rate (%), the better the preservation stability.

•Lycopene and astaxanthin

The absorbance of lycopene and astaxanthin was measured under the following measurement conditions, and the decomposition rate (%) thereof was calculated by the following Equation (A).

$$\text{Decomposition rate (\%)} = (\text{absorbance immediately after preparation} - \text{absorbance after preservation for 4 weeks at } 50°C)/\text{absorbance immediately after preparation} \times 100 \cdots \text{Equation (A)}$$

-Measurement conditions of absorbance-

Each of the samples was diluted with pure water such that the concentration of lycopene or astaxanthin in each of the samples became 0.0004% by mass, and by using an UV-VISIBLE spectrophotometer UV-2550 (manufactured by Shimadzu Corporation) and a 10 mm cell,
the absorbance at 510 nm (lycopene) and 478 nm (astaxanthin) was measured. When the concentration of lycopene or astaxanthin in each of the samples was equal to or less than 0.0004%, the sample solution was measured as is.

•Tocotrienol

Under the following conditions, the content of tocotrienol in each of the just prepared sample and the sample preserved for 4 weeks at 50°C was measured by a gradient analysis method by high performance liquid chromatography (HPLC). From the content of tocotrienol in the just prepared sample and the content of tocotrienol in the preserved sample, the decomposition rate (%) was calculated by the following Equation (B).

$$\text{Decomposition rate (\%)} = (\text{content immediately after preparation} - \text{content after preservation for 4 weeks at } 50°C)/\text{content immediately after preparation} \times 100 \cdots \text{Equation (B)}$$

~HPLC conditions~

Column: C8 5 $\mu$m 4.6 mm $\times$ 150 mm
Flow rate: 1 ml/min
Column temperature: 40°C
Detection wavelength: 297 nm
Developing solution
A: methanol/water = 75/25 (capacity ratio, containing 0.1% by capacity (volume) of acetic acid and 0.1% by capacity (volume) of triethanolamine)
B: methanol = 100 (capacity ratio, containing 0.1% by capacity (volume) of acetic acid and 0.1% by capacity (volume) of triethanolamine)

(Gradient conditions)

The gradient conditions were as shown in the following Table 3.

[Table 3]

| Time (min) | B.pump.(%) |
|---|---|
| 0.1 | 20 |
| 20 | 100 |
| 30 | 100 |
| 30.1 | 20 |
| 45 | Stop |

<Emulsion particle size>

5 ml glass vials were filled with each of the emulsion compositions of examples and comparative examples, thereby preparing samples. The volume-based average particle size (median particle size) of emulsion particles in the samples was measured by using a dynamic light scattering analyzer (trade name: FPAR-1000, manufactured by OTSUKA ELECTRONICS CO., LTD.).

The results are also shown in Table 4.

[Table 4] In Table 4 Examples 2 to 6 and 11 to 14 do not fall under the scope of the present invention.

| | | Comparative example 1 | Comparative example 2 | Comparative example 3 | Comparative example 4 | Comparative example 5 | Comparative example 6 | Comparative example 7 | Comparative example 8 | Comparative example 9 | Comparative example 10 | Example 1 | Ref. Example 2 | Ref. Example 3 | Ref. Example 4 | Ref. Example 5 | Ref. Example 6 | Example 7 | Example 8 | Example 9 | Example 10 | Ref. Example 11 | Ref. Example 12 | Ref. Example 13 | Ref. Example 14 |
|---|---|---|---|---|---|---|---|---|---|---|---|---|---|---|---|---|---|---|---|---|---|---|---|---|---|
| Emulsion containing oil-soluble antioxidant | Emulsion A | | | 0.2 | | | | | | | | | | | | | | | | | | | | | |
| | Emulsion B | 0.2 | | | 0.2 | | | | | | | | | | | | | | | | | | | | |
| | Emulsion C | | | | | 0.2 | | | | | | | | | | | | | | | | | | | |
| | Emulsion D | | | | | | 0.2 | | | | | | | | | | | | | | | | | | |
| | Emulsion E | | | | | | | 0.2 | | | | | | | | | | | | | | | | | |
| | Emulsion F | | | | | | | | 0.2 | | | | | | | | | | | | | | | | |
| | Emulsion G | | | | | | | | | | | 0.2 | | | | | | | | | | | | | |
| | Emulsion H | | | | | | | | | | | | 0.2 | | | | | | | | | | | | |
| | Emulsion I | | | | | | | | | | | | | 0.2 | | | | | | | | | | | |
| | Emulsion J | | | | | | | | | | | | | | 0.2 | | | | | | | | | | |
| | Emulsion K | | | | | | | | | | | | | | | 0.2 | | | | | | | | | |
| | Emulsion L | | | | | | | | | | | | | | | | 0.2 | | | | | | | | |
| | Emulsion M | | 0.2 | | | | | | | | | | | | | | | | 0.2 | | | 0.2 | 0.2 | 0.2 | |
| | Emulsion N | | | | | | | | | | | | | | | | | | | 0.2 | | | | | |
| | Emulsion O | | | | | | | | | | | | | | | | | | | | 0.20 | | | | |
| | Emulsion P | | | | | | | | | 0.2 | | | | | | | | | | | | | | | |
| | Emulsion Q | | | | | | | | | | | | | | | | | | | | | | | 0.2 | |
| | Emulsion R | | | | | | | | | | 0.2 | | | | | | | | | | | | | | |
| | Emulsion S | | | | | | | | | | | | | | | | | | | | | | | | 0.2 |
| Diluent | Ascorbic acid | | | | | | | | | | | | | | | | | | | | | 0.1 | | | |
| | Gallic acid | | | | | | | | | | | | | | | | | | | | | | 0.1 | | |
| | Hydroquinone | | | | | | | | | | | | | | | | | | | | | | | 0.1 | |
| | Ascorbyl Mg phosphate | | | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | | | 1.0 | 1.0 |
| | Na citrate | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 |
| | Aqueous solution of 10 mM phosphate | 98.8 | 98.8 | 97.8 | 97.8 | 97.8 | 97.8 | 97.8 | 97.8 | 97.8 | 97.8 | 97.8 | 97.8 | 97.8 | 97.8 | 97.8 | 97.8 | 97.8 | 97.8 | 97.8 | 97.8 | 98.7 | 98.7 | 98.7 | 97.8 |

| buffer with pH of 7.0 | | | | | | | | | | | | | | | | | | | | | | | | |
|---|---|---|---|---|---|---|---|---|---|---|---|---|---|---|---|---|---|---|---|---|---|---|---|---|
| Particle size immediately after preparation | 68nm | 60nm | 163nm | 66nm | 68nm | 69nm | 62nm | 161nm | 70nm | 67nm | 114nm | 67nm | 63nm | 66nm | 62nm | 62nm | 59nm | 60nm | 116nm | 57nm | 59nm | 59nm | 70nm | 66nm |
| Decomposition rate of lycopene after preservation for 4 weeks at 50°C | 32% | 34% | 31% | 29% | 30% | 29% | 27% | 30% | - | - | 17% | 17% | 14% | 10% | 8% | 13% | 8% | 12% | 18% | 3% | 13% | 14% | - | - |
| Decomposition rate of astaxanthin after preservation for 4 weeks at 50°C | - | - | - | - | - | - | - | - | 12% | - | - | - | - | - | - | - | - | - | - | - | - | - | 6% | - |
| Decomposition rate of tocotrienol after preservation for 4 weeks at 50°C | - | - | - | - | - | - | - | - | - | 9% | - | - | - | - | - | - | - | - | - | - | - | - | - | 5% |

As is evident from Table 4, in all of the emulsion compositions of examples, the decomposition rate of lycopene, astaxanthin, and tocotrienol contained as oil-soluble antioxidants preserved for 4 weeks is low. Therefore, it is understood that the emulsion compositions of examples are excellent in preservation stability.

More specifically, from the results shown in Table 4, the following facts are understood.

From the comparison between Comparative examples 1 to 8 and Examples 1 to 12 (including Reference Examples 2-6, 11 and 12), it is understood that by the combination of oil having an I/O value of equal to or less than 0.15, a water-soluble antioxidant, and emulsion particles having a particle size of equal to or less than 120 nm, the stability of lycopene is specifically improved.

From the comparison between Comparative examples 3, 9, as well as 10 and Examples 7, 13 (for reference), as well as 14 (for reference), it is understood that the combination of oil having an I/O value of equal to or less than 0.15, emulsion particles having a particle size of equal to or less than 120 nm, and a water-soluble antioxidant markedly improves the preservation stability, even when astaxanthin or tocotrienol is used as an oil-soluble antioxidant.

From the comparison between Examples 7 and 9, it is understood that if lycopene is caused to be in an amorphous state, the stability thereof is further improved.

From the comparison between Examples 7 and 10 and Reference Examples 11 and 12, it is understood that ascorbic acid and a derivative thereof are particularly preferable as a water-soluble antioxidant.

From the comparison between Examples 1 and 7, it is understood that the particle size of the emulsion particles is more preferably equal to or less than 100 nm.

From the comparison between Example 2 (for reference) and Examples 1, 3 (for reference), as well as 4 (for reference), it is understood that the I/O value of the specific oil is preferably equal to or less than 0.13.

From the comparison between Examples 3 (for reference) and 4 (for reference), it is understood that a monoester compound in which a fatty acid and a monohydric alcohol are condensed with each other is more preferable as the specific oil.

From the comparison between Examples 7 and 8, it is understood that the number of carbon atoms of the fatty acid group of polyglycerin fatty acid ester as an emulsifier is more preferably 14.

Therefore, according to the present invention, it is possible to provide an oil-in-water emulsion composition in which the decomposition of an oil-soluble antioxidant is inhibited and which is excellent in preservation stability.

Hereinafter, as an application embodiment using the emulsion composition of the present invention, examples of cosmetics (a toner, a serum, an emulsion, and a cream) as a form of an external preparation for skin will be described.

<<Tomato extract, krill extract, and hematococcus algae extract>>

Details of the tomato extract and hematococcus algae extract used for preparing each of the external preparations for skin of the examples described below are as follows.

• Tomato extract (lycopene content: 0.1% by mass, prepared by diluting 6% by mass of Lyc-O-Mato (trade name, manufactured by Sun Bright Co., Ltd.) with glycerin)

• Hematococcus algae extract (content of astaxanthin: 0.3% by mass, prepared by diluting ASTOTS-S (trade name, manufactured by Takeda Shiki co., Ltd.) with glycerin)

[Examples 15 and 16]

A toner (Example 15) and a serum (Example 16) having the composition shown in the following Table 5 were prepared.

[Table 5]

| (Total amount: 100% by mass) | | | |
|---|---|---|---|
| | | Example 15 | Example 16 |
| | | (Toner) | (Serum) |
| | Lycopene dispersion (emulsion M) | 0.04 | 0.1 |
| | Hematococcus algae extract (containing 0.3% by mass of astaxanthin) | 0.05 | - |
| | Astaxanthin dispersion (emulsion T) | - | 1 |

(continued)

| (Total amount: 100% by mass) | | Example 15 | Example 16 |
|---|---|---|---|
| | | (Toner) | (Serum) |
| Composition | Glycerin | 5 | 5 |
| | 1,3-butylene glycol | 6.5 | 6.5 |
| | Polyoxyethylene sorbitan monolauric acid ester | 1.2 | 1.2 |
| | Ethanol | 12 | 12 |
| | Tocopherol acetate | 0.001 | 0.001 |
| | Ascorbyl magnesium phosphate | 0.5 | 0.5 |
| | Xanthan gum | 0.1 | 0.1 |
| | Alcasealan | - | 0.02 |
| | Ceramide | - | 0.01 |
| | Carboxyvinyl polymer | 0.05 | - |
| | Glycacil 2000 | 0.1 | 0.1 |
| | Ethylhexylglycerin | 0.2 | 0.2 |
| | Pentylene glycol | 2 | 2 |
| | Decaglyceryl monooleate | 0.2 | - |
| | Sucrose stearic acid ester | 0.5 | - |
| | Citric acid | - | 0.05 |
| | Sodium citrate | - | 0.1 |
| | Collagen | 1 | 1 |
| | N-acetylhydroxyproline | 1 | 1 |
| | Methyl paraoxybenzoate | 0.05 | 0.05 |
| | Rose extract oil | - | 0.001 |
| | Fragrance | Appropriate amount | Appropriate amount |
| | Polyoxyethylene hydrogenated castor oil | 0.2 | 0.2 |
| | Purified water | Balance | Balance |

[Example 17]

An emulsion having the following composition was prepared (total amount: 100% by mass).

| (Component) | (% by mass) |
|---|---|
| ·Tomato extract | 0.1 |
| ·Lycopene dispersion (emulsion M) | 0.1 |
| ·Lutein | 0.01 |
| ·Hematococcus algae extract | 0.5 |
| ·Astaxanthin dispersion (emulsion T) | 0.5 |
| ·Squalane | 8.0 |
| ·Jojoba oil | 7.0 |
| ·Glyceryl para-aminobenzoate | 1.0 |
| ·Cetyl alcohol | 1.5 |

(continued)

| (Component) | (% by mass) |
|---|---|
| ·Glycerin monostearate | 2.0 |
| ·Polyoxyethylene cetyl ether | 3.0 |
| ·Polyoxyethylene sorbitan monooleate | 2.0 |
| ·1,3-Butylene glycol | 1.0 |
| ·Glycerin | 2.0 |
| ·Methylparaben | 0.04 |
| ·Sucrose stearate | 0.1 |
| ·Polyglyceryl-10 oleate | 0.1 |
| ·Tocopherol acetate | 0.01 |
| ·Phenoxyethanol | 0.2 |
| ·Collagen | 1.0 |
| ·Sodium citrate | 1.0 |
| ·Fragrance | appropriate amount |
| ·Purified water | balance |

It was confirmed that an emulsion could also be prepared even when fucoxanthin was used instead of lutein in the above composition.

[Example 18]

A cream having the following composition was prepared (total amount: 100% by mass).

| (Component) | (% by mass) |
|---|---|
| ·Lycopene dispersion (emulsion M) | 0.4 |
| ·Astaxanthin dispersion (emulsion T) | 0.2 |
| ·Cetostearyl alcohol | 3.0 |
| ·Glycerin fatty acid ester | 2.0 |
| ·Polyoxyethylene (20) sorbitan monooleate | 1.0 |
| ·Sorbitan monostearate | 1.0 |
| ·N-stearoyl-N-methyltaurine sodium | 0.5 |
| ·Vaseline | 5.0 |
| ·Lecithin | 0.5 |
| ·Dimethylpolysiloxane (100 mPa·s) | 3.0 |
| ·Glyceryl tri-2-ethylhexanoate | 20.0 |
| ·Lactic acid | 1.0 |
| ·Magnesium ascorbyl phosphate | 0.5 |
| ·Dipropylene glycol | 10.0 |
| ·Sodium citrate | 0.5 |
| ·Titanium oxide | 0.1 |
| ·Fragrance | appropriate amount |
| ·Disodium edetate | 0.03 |
| ·Ethyl para-oxybenzoate | 0.05 |
| ·Purified water | balance |

[Example 19]

A jelly-type serum having the following composition was prepared according to a common method (total amount: 100% by mass)

| (Component) | (% by mass) |
|---|---|
| ·Lycopene dispersion (emulsion M) | 0.01 |

(continued)

| (Component) | (% by mass) |
|---|---|
| ·Astaxanthin dispersion (emulsion T) | 0.02 |
| ·Tocopherol | 0.01 |
| ·Ceramide III, VI | 0.01 |
| ·(PEG-240/decyl tetradeses-20/HDI) copolymer | 0.5 |
| ·Hydrolyzed collagen | 1.0 |
| ·Acetyl hydroxyproline | 1.0 |
| ·Ethyl hexyl glycerin | 0.05 |
| ·Oleic acid | 0.05 |
| ·1,3-Butylene glycol | 1.0 |
| ·Glycerin | 2.0 |
| ·Methylparaben | 0.2 |
| ·Phenoxyethanol | 0.2 |
| ·Collagen | 1.0 |
| ·Sodium citrate | 1.0 |
| ·Damask rose oil | appropriate amount |
| ·Fragrance | appropriate amount |
| ·Purified water | balance |

## Claims

1.  An oil-in-water emulsion composition comprising:

    an oil phase containing octyldodecyl myristate as an oil having an I/O value of equal to or less than 0.15 and lycopene as an oil-soluble antioxidant;
    a water phase containing at least one kind of compound selected from the group consisting of ascorbic acid, a derivative thereof selected from the group consisting of sodium L-ascorbate, potassium L-ascorbate, calcium L-ascorbate, L-ascorbic acid phosphoric acid ester, a magnesium salt of L-ascorbic acid phosphoric acid ester, L-ascorbic acid sulfuric acid ester, a disodium salt of L-ascorbic acid sulfuric acid ester, L-ascorbic acid stearic acid ester, L-ascorbic acid 2-glucoside, L-ascorbic acid palmitic acid ester, L-ascorbyl tetraisopalmitate, and fatty acid esters of ascorbic acid such as L-ascorbyl stearic acid ester, L-ascorbyl tetraisopalmitic acid ester, and L-ascorbyl palmitic acid ester, and a salt of these as a water-soluble antioxidant, and a polyglycerin fatty acid ester having HLB of equal to or greater than 10 as an emulsifier, wherein the polyglycerin fatty acid ester having HLB of equal to or greater than 10 comprises at least one compound selected from the group consisting of decaglycerin monooleic acid ester, decaglycerin monostearic acid ester, decaglycerin monopalmitic acid ester, decaglycerin monomyristic acid ester and decaglycerin monolauric acid ester, wherein a content of the octyldodecyl myristate in the oil phase is in a range of 85% by mass to 99% by mass, and
    wherein the average particle size of emulsion particles is equal to or less than 120 nm.

2.  The emulsion composition according to claim 1, wherein the lycopene is contained in an amorphous state.

## Patentansprüche

1.  Öl-in-Wasser Emulsionszusammensetzung umfassend:

    eine Ölphase enthaltend Octyldodecylmyristat als ein Öl, das einen I/O Wert von gleich oder weniger als 0.15 aufweist, und Lycopen als ein öllösliches Antioxidans;
    eine Wasserphase enthaltend mindestens einen Typ an Verbindung, ausgewählt aus der Gruppe bestehend aus Ascorbinsäure, einem Derivat davon ausgewählt aus der Gruppe bestehend aus Natrium-L-ascorbat, Kalium-L-ascorbat, Calcium-L-ascorbat, L-Ascorbinsäurephosphorsäureester, einem Magnesiumsalz von L-Ascorbinsäurephosphorsäureester, L-Ascorbinsäureschwefelsäureester, einem Dinatriumsalz von L-Ascorbinsäureschwefelsäureester, L-Ascorbinsäurestearinsäureester, L-Ascorbinsäure-2-glucosid, L-Ascorbinsäurepalmitinsäureester, L-Ascorbyltetraisopalmitat und Fettsäureestern von Ascorbinsäure, wie L-Ascorbylstearinsäureester, L-Ascorbyltetraisopalmitinsäureester, and L-Ascorbylpalmitinsäureester, und einem Salz von diesen als ein wasserlösliches Antioxidans, und einen Polyglycerinfettsäureester mit einem HLB von gleich oder größer als 10 als ein Emulgator, wobei der Polyglycerinfettsäureester mit einem HLB von gleich oder größer als 10 mindestens eine Verbindung, ausgewählt aus der Gruppe bestehend aus Decaglycerinmonoölsäureester, Decaglycerinmonostearinsäureester, Decaglycerinmonopalmitinsäureester, Decaglycerinmonomyristinsäureester und Decaglycerinmonolaurinsäureester umfasst, wobei ein Gehalt an dem Octyldodecylmyristat in der Ölphase in einem Bereich von 85 Masse% bis 99 Masse% liegt, und
wobei die durchschnittliche Partikelgröße der Emulsionspartikel gleich oder weniger als 120 nm ist.

2. Emulsionszusammensetzung nach Anspruch 1, wobei das Lycopen in einem amorphen Zustand enthalten ist.

**Revendications**

1. Composition d'émulsion huile dans eau, comprenant :

une phase huileuse contenant du myristate d'octyldodécyle comme huile présentant une valeur I/O inférieure ou égale à 0,15 et du lycopène comme antioxydant liposoluble ;
une phase aqueuse contenant au moins un type de composé sélectionné parmi le groupe consistant en l'acide ascorbique, un dérivé de celui-ci sélectionné parmi le groupe consistant en le L-ascorbate de sodium, le L-ascorbate de potassium, le L-ascorbate de calcium, un ester d'acide phosphorique d'acide L-ascorbique, un sel de magnésium d'ester d'acide phosphorique d'acide L-ascorbique, un ester d'acide sulfurique d'acide L-ascorbique, un sel disodique d'ester d'acide sulfurique d'acide L-ascorbique, un ester d'acide stéarique d'acide L-ascorbique, un 2-glucoside d'acide L-ascorbique, un ester d'acide palmitique d'acide L-ascorbique, un tétraisopalmitate de L-ascorbyle, et des esters d'acides gras d'acide ascorbique tels que l'ester d'acide stéarique de L-ascorbyle, l'ester d'acide tétraisopalmitique de L-ascorbyle, l'ester d'acide palmitique de L-ascorbyle, et un sel de ceux-ci comme antioxydant hydrosoluble, et un ester d'acide gras de polyglycérine présentant une valeur HLB (balance hydrophile-lipophile) supérieure ou égale à 10 comme émulsifiant, dans laquelle l'ester d'acide gras de polyglycérine présentant une valeur HLB supérieure ou égale à 10 comprend au moins un composé sélectionné parmi le groupe consistant en un ester d'acide mono-oléique de décaglycérine, un ester d'acide mono-stéarique de décaglycérine, un ester d'acide mono-palmitique de décaglycérine, un ester d'acide mono-myristique de décaglycérine, et un ester d'acide mono-laurique de décaglycérine, dans laquelle une teneur du myristate d'octyldodécyle dans la phase huileuse est comprise dans la plage allant de 85% en masse à 99 % en masse, et
dans laquelle la taille de particules moyennes des particules d'émulsion est inférieure ou égale à 120 nm.

2. Composition d'émulsion selon la revendication 1, dans laquelle le licopène est contenu dans un état amorphe.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4717790 B
- JP 2011241177 A
- JP 2002078447 A

### Non-patent literature cited in the description

- **KODA YOSHIO.** Organic Conceptual Diagram. SAN-KYO PUBLISHING Co., Ltd, 1984
- Theory and Practice of Antioxidants. Kajimoto, Sanshobo, 1984
- **SARUWATARI ; NISHINO ; TABATA.** Handbook of Antioxidants. TAISEISHA, LTD, 1976